# FASCICULE DE BREVET EUROPEEN

(11) **EP 3 492 548 B1**
(45) Date de publication et mention de la délivrance du brevet: **24.08.2022**
(21) Numéro de dépôt: 18207547.3
(22) Date de dépôt: 06.12.2013
(51) Int. Cl.: C09K 5/04

(54) **COMPOSITION COMPRENANT DU 2,3,3,3-TETRAFLUOROPROPENE**
ZUSAMMENSETZUNG, DIE 2,3,3,3-TETRAFLUORPROPEN ENTHÄLT
COMPOSITION COMPRISING 2,3,3,3-TETRAFLUOROPROPENE

(30) Priorité: 26.12.2012 FR 1262766
(43) Date de publication de la demande: 05.06.2019
(62) Demande divisionnaire de: 13821859.9
(73) Titulaire: Arkema France, 92700 Colombes (FR)
(72) Inventeur: COLLIER, Bertrand, 92705 COLOMBES (FR); DEUR-BERT, Dominique, 92705 COLOMBES (FR); WENDLINGER, Laurent, 69491 PIERRE-BENITE CEDEX (FR)
(74) Mandataire: Arkema Patent

(56) Documents cités:
- EP-A1- 2 837 613
- WO-A1-2012/098420
- WO-A2-2009/125200
- WO-A2-2009/137658
- WO-A2-2013/032908
- US-A1- 2007 112 227
- US-A1- 2007 112 229
- US-A1- 2011 031 436

## Description

La présente invention concerne des compositions comprenant du 2,3,3,3-tetrafluoropropène, utiles dans de nombreux domaines d'applications tels que réfrigération, agent d'expansion, solvants et aérosols.

Un paramètre très important dans le choix d'une composition utile dans les domaines de la réfrigération, de l'air-conditionné et des pompes à chaleurs est son impact sur l'environnement.

La fabrication du 2,3,3,3-tetrafluoropropène (HFO-1234yf) s'accompagnant d'une multitude de sous-produits, ayant un point d'ébullition proche du HFO-1234yf, conduit à des étapes de purification assez complexes et coûteuses. La difficulté rencontrée au cours de la purification du HFO-1234yf implique généralement une perte conséquente en produit recherché. De plus, ces sous-produits peuvent former des compositions binaires ou ternaires azéotropiques avec le HFO-1234yf, rendant la séparation par distillation simple impossible.

Le document US 2011/031436 décrit une composition comprenant HFO-1234yf et au moins un composé additionnel sélectionné parmi le groupe consistant en HFO-1234ze, HFO-1243zf, HCFC-243 db, HCFC-244 db, HFC-245cb, HFC-245fa, HCFO-1233xf, HCFO-1233zd, HCFC-253fb, HCFC-234ab, HCFC-243fa, ethylene, HFC-23, CFC-13, HFC-143a, HFC-152a, HFC-236fa, HCO-1130, HCO-1130a, HFO-1336, HCFC-133a, HCFC-254fb, HCFC-1131, HFO-1141, HCFO-1242zf, HCFO-1223xd, HCFC-233ab, HCFC-226ba, and HFC-227ca. Le document WO 2021/098420 décrit décrit un procédé catalytique de fluoration en phase gazeuse de 1,1,1,2,3-pentachloropropane et/ou 1,1,2,2,3-pentachloropropane en 2,3,3,3-tétrafluoropropène. US 2007/112227 décrit une composition comprenant 23 mol. % de HFO-1234yf et des impuretés. US 2007/112229 décrit la production de HFO-1234yf à partir de HFC-235cb. EP 2 837 613 décrit une composition azéotropique HFO-1234yf et HCC-40. Le document WO 2009/125200 décrit un procédé de préparation du HCFC-243db par mise en contact entre le HFO-1243zf et du chlore en présence d'un catalyseur de type carbone activé, alumine et/ou un oxyde d'un métal de transition. WO 2009/137658 décrit des compositions comprenant HCFC-243db, HCFO-1233xf, HCFC-244bb et/ou HFO-1234yf et au moins un composé additionnel. WO 2013/032908 décrit des compositions comprenant HFO-1234yf. US 2012/0065437 décrit une composition comprenant 99,96% de HFO-1234yf produite à partir du HCFC-244bb en présence d'un catalyseur CsCl/MgF2.

La présente invention a pour objet une composition comprenant le composé HFO-1234yf, le HFO-1243zf et HCFC-115 ; le composé HFO-1234yf représentant au moins 99% en poids dans la composition.

De préférence, la totalité des composés additionnels représente au plus 1 % en poids de la composition comprenant le HFO-1234yf et avantageusement au plus 0,5 % en poids.

Les composés tels que HCFC-115, HFC-152a et HCC-40 ont un point d'ébullition particulièrement proche de celui de HFO-1234yf.

Avantageusement, le HCFC-115 et/ou HFC-152a et/ou HCC-40 lorsqu'il(s) est (sont) présent(s) dans la composition représente au plus 500 ppm et particulièrement préféré représente au plus 50 ppm.

Selon ce mode de réalisation, la composition peut comprendre en outre au moins un composé choisi parmi le HCFC-240db, HCFO-1233xf, HCFC-243db, HCFO-1233zd, HCFC-114a, HCFC-122, HCFC-123, HCFC-124, HCFC-124a, HFC-125, HCFC-133a, HCFC-142, HCFC-143, HCFC-243ab, HCFC-244eb, HFC-281ea, HCO-1110, HCFO-1111, HCFO-1113, HCFO-1223xd, HCFO-1224xe.

Quelque soit le mode de réalisation, la totalité des composé(s) additionnel(s) représente au plus 1 % en poids de la composition comprenant le HFO-1234yf et avantageusement au plus 0,5 % en poids.

A titre d'exemple on peut citer notamment les composés suivants dont les acronymes représentent :
- HCFC-240db : 1,1,1,2,3-pentachloropropane ou CCl₃-CHCl-CH₂Cl
- HCFO-1233xf : 3,3,3-trifluoro-2-chloropropene ou CF₃-CCl=CH₂
- HCFC-243db : 1,1,1-trifluoro-2,3-dichloropropane ou CF₃-CHCl-CH₂Cl
- HCFO-1233zd : E/Z-3,3,3-trifluoro-1-chloropropene ou CF₃-CH=CHCl
- HCC-40 : chloromethane ou CH₃Cl
- HCFC-114a : 1,1,1,2-tetrafluoro-2,2-dichloroethane ou CF₃-CCl₂F
- HCFC-115 : 1,1,1,2,2-pentafluoro-2-chloroethane CF₃-CClF₂
- HCFC-122 : 1,1,2-trichloro-2,2-difluoroethane ou CHCl₂-CClF₂
- HCFC-123 : 1,1,1-trifluoro-2,2-dichloroethane ou CF₃-CHCl₂
- HCFC-124 : 1,1,1,2-tetrafluoro-2-chloroethane ou CF₃-CHClF
- HCFC-124a : 1,1,2,2,-tetrafluoro-2-chloroethane ou CHF₂-CClF₂
- HFC-125 : 1,1,1,2,2,-pentafluoroethane ou CF₃-CHF₂
- HCFC-133a : 1,1,1-trifluoro-2-chloroethane ou CF₃-CH₂Cl
- HCFC-142 : 1,1-difluoro-2-chloroethane ou CHF₂-CH₂Cl
- HCFC-143 : 1,1,2-trifluoroethane ou CHF₂-CH₂F
- HFC-152a : 1,1-difluoroethane ou CHF₂-CH₃
- HCFC-243ab : 1,1,1-trifluoro-2,2-dichloropropane ou CF₃-CCl₂- CH₃
- HCFC-244eb : 1,1,1,2-tetrafluoro-3-chloropropane ou CF₃-CHF-CH₂Cl
- HFC-281ea : 2-fluoropropane ou CH₃-CFH-CH₃
- HCO-1110 : 1,1,2,2-tetrachloroethylene ou CCl₂=CCl₂
- HCFO-1111 : 1,1,2-trichloro-2-fluoroethylene ou CCl₂=CClF
- HCFO-1113 : 1,1,2-trifluoro-2-chloroethylene ou CF₂=CClF
- HCFO-1223xd : E/Z-3,3,3-trifluoro-1,2-dichloropropene ou CF₃-CCl=CHCl
- HCFO-1224xe : E/Z-1,3,3,3-tetrafluoro-2-chloropropene ou CF₃-CCl=CHF
- HFO-1234ze : E/Z-1,3,3,3-tetrafluoropropene ou CF₃-CH=CHF
- HFC-245cb : 1,1,1,2,2-pentafluoropropane ou CF₃-CF₂-CH₃
- HFC-245eb : 1,1,1,2,3-pentafluoropropane ou CF₃-CHF-CH₂F
- HFC-245fa : 1,1,1,3,3-pentafluoropropane ou CF₃-CH₂-CHF₂
- HFC-23 : trifluoromethane ou CHF₃
- HFC-134a : 1,1,1,2-tetrafluoroethane ou CF₃-CH₂F
- HFC-143a : 1,1,1-trifluoroethane ou CF₃-CH₃
- HFC-236fa : 1,1,1,3,3,3-hexafluoropropane ou CF₃-CH₂-CF₃
- HCFC-244bb : 1,1,1,2-tetrafluoro-2-chloropropane ou CF₃-CFCl-CH₃
- HCFC-244db : 1,1,1,3-tetrafluoro-2-chloropropane ou CF₃-CHCl-CH₂F
- HFO-1132a : 1,2-difluoroethylene ou CHF=CHF
- HFO-1223 : 3,3,3-trifluoropropyne ou CF₃-C≡CH
- HFO-1225zc : E/Z-1,1,3,3,3-pentafluoropropene ou CF₃-CH=CF₂
- HFO-1225ye : E/Z-1,2,3,3,3-pentafluoropropene ou CF₃-CF=CHF
- HCFO-1232xf : 3,3-difluoro-1,3-dichloropropene ou CClF₂-CCl=CH₂
- HFO-1243zf : 3,3,3-trifluoropropene ou CF₃-CH=CH₂

Selon un mode de mise en œuvre plus particulier, la composition selon l'invention peut comprendre un mélange ternaire, par exemple un mélange choisi parmi:
- HFO-1234yf, HFO-1243zf, HCFC-115

Les compositions préférées et /ou précitées peuvent en outre comprendre au moins un autre composé additionnel choisi parmi HCFC-240db, HCFO-1233xf, HCFC-243db, HCFO-1233zd, HCFC-114a, HCFC-122, HCFC-123, HCFC-124, HCFC-124a, HFC-125, HCFC-133a, HCFC-142, HCFC-143, HCFC-243ab, HCFC-244eb, HFC-281ea, HCO-1110, HCFO-1111, HCFO-1113, HCFO-1223xd, HFC-245eb, HFC-245fa, HFC-23, HFC-143a, HFC-236fa, HCFC-244bb, HCFC-244db, HFO-1132a, HFO-1223, HFO-1225zc, HFO-1225ye, HCFO-1232xf et HCFO-1224xe.

Quelque soit le mode de réalisation, le composé HFO-1234yf représente de préférence au moins 99 % en poids dans la composition et avantageusement au moins 99,5% en poids.

La composition selon l'invention peut être obtenue à partir du HCC-240db et en faisant appel à une ou plusieurs étapes réactionnelles.

Ainsi, le HCC-240db peut être soumis à une étape réactionnelle en phase gaz avec un agent de fluoration, de préférence I'HF anhydre, pour donner directement le HFO-1234yf, éventuellement accompagné de produits intermédiaires choisis parmi le HCFO-1233xf, HCFC-243db et HCFO-1233zd. La réaction de fluoration peut être mise en œuvre en présence de catalyseur et de préférence à une température comprise entre 100 °C et 500 °C, plus préférentiellement comprise entre 200 et 450 °C. Après séparation du HFO-1234yf, notamment par décantation suivie de distillation, le(s) produit(s) intermédiaire(s) et le cas échéant le HCC-240db non réagi peuvent ensuite être recyclés à l'étape réactionnelle.

La composition selon l'invention peut également être obtenue à partir du HCC-240db au moyen d'au moins deux étapes réactionnelles. La première étape consiste en général à soumettre le HCC-240db à une réaction en phase gaz avec un agent de fluoration, de préférence de I'HF anhydre, pour donner au moins un produit intermédiaire tel que le HCFO-1233xf. Dans une deuxième étape, le produit intermédiaire réagit avec un agent de fluoration, de préférence de I'HF anhydre, pour donner une composition comprenant le HFO-1234yf et au moins un composé additionnel tel que décrit ci-dessus. A l'issue de la deuxième étape, la composition comprenant le HFO-1234yf et au moins un composé additionnel est soumise à une étape de séparation et/ou purification.

Les deux étapes peuvent être mise en œuvre en présence d'un catalyseur, le catalyseur pouvant être identique ou différent. Ces étapes peuvent être mise en œuvre dans un même réacteur lorsque la réaction effectuée en phase gazeuse. Dans ce cas, le réacteur peut comprendre un lit catalytique supérieur différent de celui du lit inférieur.

Lorsque la préparation est effectuée à l'aide de deux étapes réactionnelles, la température de réaction de la première étape est en général inférieure à celle de la deuxième étape et est de préférence comprise entre 100 °C et 500 °C, plus préférentiellement comprise entre 200 et 450 °C.

En cas de besoin, après l'étape de séparation et/ou purification précitée, le flux comprenant du HFO-1234yf peut être soumis à une étape de distillation azéotropique et/ou d'adsorption par du charbon actif et/ou tamis moléculaire ou à une étape de photochloration.

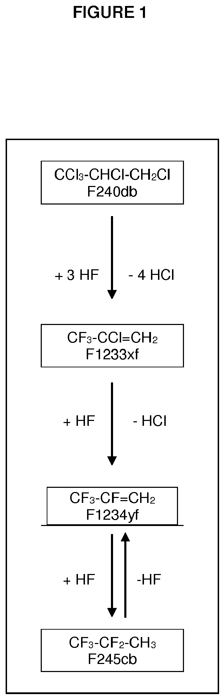

Selon un mode de réalisation de l'invention, la composition est obtenue par une série de réactions (figure 1). La figure 1 illustre une série de réactions pour la production de HFO-1234yf. La série de réaction commence par une réaction d'hydrofluoration de HCC-240db, avec du fluorure d'hydrogène en HCFO-1233xf. Le composé HCFO-1233xf peut subir à son tour une réaction d'hydrofluoration en HFO-1234yf. Le composé HFO-1234yf peut à son tour subir une réaction d'hydrofluoration en HFC-245cb. De multitudes de produits peuvent être obtenus par des réactions parallèles à cette série de réactions par exemple par des réactions d'isomérisation, d'addition d'HCI et de chloration.

Les réactions décrites précédemment sont préférentiellement réalisées, en présence de catalyseur de fluoration supporté ou non, de préférence activé en présence de fluorure d'hydrogène et/ou d'air, comprenant des oxydes de chrome et éventuellement un co-catalyseur à base par exemple de nickel, de zinc, de titane, de magnésium et d'étain.

La(les) réaction(s) d'hydrofluoration peut/peuvent être conduite(s) en phase gazeuse, éventuellement en présence d'une quantité suffisante d'oxygène.

La production de HFO-1234yf peut être conduite dans un ou plusieurs réacteurs en séries. L'alimentation en HCC-240db peut être placée à l'entrée d'un des réacteurs ou à l'entrée du premier réacteur en série, ou à chacune des entrées de chacun des réacteurs en série.

La(les) réaction(s) d'hydrofluoration peut(peuvent) être conduite(s) en continu, semi-continu.

La production de HFO-1234yf peut être préférentiellement conduite à une pression absolue comprise entre 0,1 et 50 bars, plus préférentiellement entre 0,3 et 15 bars.

Le temps de contact dans le réacteur est compris entre 1 et 100 secondes, préférentiellement compris entre 5 et 50 secondes.

Le ratio molaire entre le fluorure d'hydrogène et les composés organiques sont compris entre 4:1 et 100:1, de préférence entre 5:1 et 50:1.

Les compositions décrites selon l'invention peuvent contenir HF, HCI et des gaz inertes (azote, oxygène, dioxyde de carbone, monoxyde de carbone, etc).

Des étapes de neutralisation/élimination d'HF et HCI peuvent être également conduites. Les compositions décrites selon l'invention peuvent également ne pas contenir ou contenir des traces d'HF et/ou d'HCI.

En présence d'un large excès d'HF mis en jeu dans l'(les) étape(s) réactionnelle(s), la fabrication du HFO-1234yf peut comprendre, au moins une étape de distillation pour récupérer une partie d'HF qui peut être recyclée à l'(les) étape(s) réactionnelle(s).

Les compositions selon l'invention sont utiles dans de nombreux domaines d'applications, notamment en tant que fluide de transfert de chaleur, propulseurs, agents moussants, agents gonflants, diélectriques gazeux, milieu de polymérisation ou monomère, fluides supports, agents pour abrasifs, agents de séchage et fluides pour unité de production d'énergie.

En présence d'un large excès d'HF mis en jeu dans l'(les) étape(s) réactionnelle(s), la fabrication du HFO-1234yf peut comprendre, au moins une étape de distillation pour récupérer une partie d'HF qui peut être recyclée à l'(les) étape(s) réactionnelle(s).

Les compositions selon l'invention sont utiles dans de nombreux domaines d'applications, notamment en tant que fluide de transfert de chaleur, propulseurs, agents moussants, agents gonflants, diélectriques gazeux, milieu de polymérisation ou monomère, fluides supports, agents pour abrasifs, agents de séchage et fluides pour unité de production d'énergie.

## Revendications

1. Composition comprenant le composé HFO-1234yf, le HFO-1243zf et HCFC-115 ; le composé HFO-1234yf représentant au moins 99 % en poids dans la composition.

2. Composition selon la revendication précédente **caractérisée en ce qu'**elle comprend en outre au moins un autre composé additionnel choisi parmi HCFC-240db, HCFO-1233xf, HCFC-243db, HCFO-1233zd, HCFC-114a, HCFC-122, HCFC-123, HCFC-124, HCFC-124a, HFC-125, HCFC-133a, HCFC-142, HCFC-143, HCFC-243ab, HCFC-244eb, HFC-281ea, HCO-1110, HCFO-1111, HCFO-1113, HCFO-1223xd, HFC-245eb, HFC-245fa, HFC-23, HFC-143a, HFC-236fa, HCFC-244bb, HCFC-244db, HFO-1132a, HFO-1223, HFO-1225zc, HFO-1225ye, HCFO-1232xf et HCFO-1224xe.

## Patentansprüche

1. Zusammensetzung, umfassend HFO-1234yf, HFO-1243zf und HFCKW-115; wobei die Verbindung HFO-1234yf mindestens 99 Gew.-% in der Zusammensetzung ausmacht.

2. Zusammensetzung nach dem vorhergehenden Anspruch, **dadurch gekennzeichnet, dass** sie außerdem mindestens eine zusätzliche Verbindung, die aus HFCKW-240db, HCFO-1233xf, HFCKW-243db, HCFO-1233zd, HFCKW-114a, HFCKW-122, HFCKW-123, HFCKW-124, HFCKW-124a, HFKW-125, HFCKW-133a, HFCKW-142, HFCKW-143, HFCKW-243ab, HFCKW-244eb, HFKW-281ea, HCO-1110, HCFO-1111, HCFO-1113, HCFO-1223xd, HFKW-245eb, HFKW-245fa, HFKW-23, HFKW-143a, HFKW-236fa, HFCKW-244bb, HFCKW-244db, HFO-1132a, HFO-1223, HFO-1225zc, HFO-1225ye, HCFO-1232xf und HCFO-1224xe ausgewählt ist, umfasst.

## Claims

1. Composition comprising the compound HFO-1234yf, HFO-1243zf and HCFC-115; the compound HFO-1234yf representing at least 99% by weight in the composition.

2. Composition according to the preceding claim, **characterized in that** it further comprises at least one other additional compound chosen from HCFC-240db, HCFO-1233xf, HCFC-243db, HCFO-1233zd, HCFC-114a, HCFC-122, HCFC-123, HCFC-124, HCFC-124a, HFC-125, HCFC-133a, HCFC-142, HCFC-143, HCFC-243ab, HCFC-244eb, HFC-281ea, HCO-1110, HCFO-1111, HCFO-1113, HCFO-1223xd, HFC-245eb, HFC-245fa, HFC-23, HFC-143a, HFC-236fa, HCFC-244bb, HCFC-244db, HFO-1132a, HFO-1223, HFO-1225zc, HFO-1225ye, HCFO-1232xf and HCFO-1224xe.
